# EUROPEAN PATENT APPLICATION

(11) **EP 3 339 862 A1**
(43) Date of publication of application: **27.06.2018**
(21) Application number: 16836983.3
(22) Date of filing: 03.08.2016
(51) Int. Cl.: G01N 33/531, G01N 33/543

(54) **IMMUNOLOGICAL DETECTION METHOD AND TEST STRIP USED THEREFOR**

(30) Priority: 17.08.2015 JP 2015160311
(71) Applicant: Sekisui Medical Co., Ltd., Tokyo 103-0027 (JP)
(72) Inventor: OCHIAI, Yasushi, Tokyo 103-0027 (JP); NISHITANI, Kimiyoshi, Tokyo 103-0027 (JP)
(74) Representative: Hoffmann Eitle
(86) International application number: PCT/JP2016/072774
(87) International publication number: WO 2017/029992

(57) **Abstract**

A problem is to provide a method of inhibiting nonspecific reaction unique to feces in a detection method using immunochromatography developing a fecal sample in an insoluble membrane so as to detect an analyte in the sample, and an immunochromatographic test strip used therefor. The present invention provides a method of performing an immunochromatographic reaction in the presence of an anti-IgA antibody so as to detect an analyte in a fecal sample without being affected by nonspecific reaction, and an immunochromatographic test strip used therefor.

## Description

### TECHNICAL FIELD

The present invention relates to an inhibitor of nonspecific reaction in an immunoreaction caused by a fecal sample (feces-derived sample), as well as a nonspecific reaction-inhibition method and an immunological detection method using the same.

Particularly, the present invention relates to an inhibitor of nonspecific reaction in an immunoreaction using immunochromatography, as well as a nonspecific reaction-inhibition method, a detection method, and an immunochromatographic test strip using the same.

### BACKGROUND ART

Immunological detection methods are widespread because detection can be achieved with high specificity and sensitivity. Particularly, detection methods using immunochromatography are widespread due to convenience. Examples of samples subjected to the detection include urine, feces, saliva, blood, serum, etc., which are suitable as clinical test samples and widely used as diagnostically useful samples.

To detect an analyte contained in these samples by using immunochromatography, various detection-interfering substances contained in the samples must be eliminated. The interfering substances include a substance directly interfering with development of a sample by immunochromatography, i.e., a substance causing clogging of an insoluble membrane for development, and a substance showing a color reaction despite the absence of an analyte, i.e., a substance causing a so-called nonspecific reaction.

In general, to eliminate a substance causing clogging in an insoluble membrane, it is known in the art that the function of separating such a substance is allocated to a sample pad and, if whole blood is used as a sample, a blood separation membrane is disposed downstream of a sample addition portion so as to remove blood cells. However, the causes for such nonspecific reaction are not clarified although various causes are possible and, therefore, countermeasures are not established at present.

Gelatin, casein, bovine serum albumin, synthetic polymers, etc. are generally known as reagents for blocking such nonspecific reaction in an antigen-antibody reaction, and methods of allowing these substances to coexist in a reaction system using immunochromatography are known (Patent Documents 1 and 2).

Although various methods for eliminating the influence of nonspecific reaction are known as described above, it is unknown whether the methods have an effect on inhibition of nonspecific reaction caused by a fecal sample.

Patent Document 3 describes a problem in the case of using feces as a specimen that methods such as ELISA using feces as a specimen have low detection sensitivity, and frequently cause nonspecific reaction, making it difficult to judge the result. Patent Document 3 discloses a method of inhibiting nonspecific reaction by using a specimen diluent of pH 9.0 to 10.0 when detecting norovirus or sapovirus by using immunochromatography, so as to solve this problem. However, this method has a problem in terms of handling because it is necessary to make the diluent alkaline, which is not common for a specimen diluent.

### CITATION LIST

### PATENT LITERATURE

Patent Document 1: Japanese Laid-Open Patent Publication No. 2003-344406
Patent Document 2: Japanese Laid-Open Patent Publication No. 2002-148266
Patent Document 3: Japanese Laid-Open Patent Publication No. 2004-301684

### SUMMARY OF INVENTION

### TECHNICAL PROBLEM

A problem to be solved by the present invention is to provide an inhibitor of nonspecific reaction which inhibits nonspecific reaction unique to feces in a method of immunologically detecting an analyte in a fecal sample, as well as a nonspecific reaction-inhibition method and an immunological detection method using the same.

Particularly, a problem is to provide an inhibitor of nonspecific reaction in a method using immunochromatography developing a fecal sample in an insoluble membrane so as to detect an analyte in the sample, a nonspecific reaction-inhibition method, an immunological detection method, and an immunochromatographic test strip.

### SOLUTION TO PROBLEM

Although blocking agents having hitherto commonly been used have effects on a certain proportion of samples when an analyte in fecal samples is immunologically detected, the present inventors have found the existence of a unique nonspecific-reaction causative substance in samples, i.e., fecal samples, in which nonspecific reaction is not inhibited by any of the blocking agents.

As a result of searching for a substance having an effect of inhibiting nonspecific reaction on such a unique causative substance, the present inventors have surprisingly found that, by performing a reaction of an immunoassay under the condition where an anti-IgA antibody is present, nonspecific reaction can be inhibited to prevent a false positive reaction so as to accurately detect an analyte, thereby completing the present invention.

Therefore, the present invention has the following configurations.
<1> A method of immunologically detecting an analyte in a fecal sample, wherein an immunoreaction is performed in the presence of an anti-IgA antibody.
<2> The method according to item <1> above, wherein the immunoreaction is performed in the presence of the anti-IgA antibody and an anti-IgM antibody.
<3> The method according to item <1> above, wherein the method is a detection method using immunochromatography developing the fecal sample in an insoluble membrane so as to detect a complex between the analyte in the sample and a conjugate, and wherein the immunological detection method includes the steps of:
   (a) bringing the sample into contact in the presence of the anti-IgA antibody with the conjugate in which an antibody or antigen immunologically reactive with the analyte is immobilized on a label, so as to form the complex between the analyte in the sample and the conjugate; and
   (b) developing the complex in the insoluble membrane so as to detect the complex in a detecting portion in which an antibody or antigen immunologically reactive with the analyte is immobilized.
<4> The method according to item <3> above, wherein the step of (a) above is a step of bringing the sample into contact in the presence of both the anti-IgA antibody and an anti-IgM antibody with the conjugate in which an antibody or antigen immunologically reactive with the analyte is immobilized on a label, so as to form the complex between the analyte in the sample and the conjugate.
<5> A method of inhibiting nonspecific reaction in a method of immunologically detecting an analyte in a fecal sample, wherein an immunoreaction is performed in the presence of an anti-IgA antibody.
<6> The method according to item <5> above, wherein the immunoreaction is performed in the presence of the anti-IgA antibody and an anti-IgM antibody.
<7> The method according to item <5> above, wherein the method is a method of inhibiting nonspecific reaction in detection using immunochromatography developing a fecal sample in an insoluble membrane so as to detect a complex between the analyte in the sample and a conjugate, and wherein the immunological detection method includes the steps of:
   (a) bringing the sample into contact in the presence of the anti-IgA antibody with the conjugate in which an antibody or antigen immunologically reactive with the analyte is immobilized on a label, so as to form the complex between the analyte in the sample and the conjugate; and
   (b) developing the complex in the insoluble membrane so as to detect the complex in a detecting portion in which an antibody or antigen immunologically reactive with the analyte is immobilized.
<8> The method according to item <7> above, wherein the step of (a) above is a step of bringing the sample into contact in the presence of both the anti-IgA antibody and an anti-IgM antibody with the conjugate in which an antibody or antigen immunologically reactive with the analyte is immobilized on a label, so as to form the complex between the analyte in the sample and the conjugate.
<9> An immunochromatographic test strip for developing a fecal sample in an insoluble membrane and detecting a complex between an analyte in the sample and a conjugate, the test strip having the conjugate in which an antibody or antigen immunologically reactive with the analyte is immobilized on a label, the test strip comprising:
   (1) a sample pad having a sample supply portion and an anti-IgA antibody retained in an elutable manner; and
   (2) an insoluble membrane having a detecting portion on which an antibody or antigen immunologically reactive with the analyte is immobilized for detecting the complex between the analyte in the sample and the conjugate.
<10> The test strip according to item <9> above, comprising a conjugate pad having the conjugate retained in an elutable manner.
<11> The test strip according to item <9> or <10> above, wherein the sample pad further has an anti-IgM antibody retained in an elutable manner.
<12> An inhibitor for a fecal sample of nonspecific reaction in an immunoreaction comprising an anti-IgA antibody as an active ingredient.
<13> The inhibitor of nonspecific reaction according to item <12> above, further comprising an anti-IgM antibody.

### ADVANTAGEOUS EFFECTS OF INVENTION

By performing an immunoreaction in the presence of an anti-IgA antibody, nonspecific reaction caused by a fecal sample can be inhibited and an analyte in the sample can accurately be detected. Particularly, by using immunochromatography as the immunoreaction, the analyte in the fecal sample can easily and accurately be detected.

### BRIEF DESCRIPTION OF DRAWINGS

[Fig. 1] Fig. 1 is a schematic configuration diagram of a test strip of the present invention.
[Fig. 2] Fig. 2 is a graph of results of examination of an influence on virus detection sensitivity when an inhibitor of nonspecific reaction of the present invention is added.

### DESCRIPTION OF EMBODIMENTS

### (Anti-IgA antibody)

The anti-IgA antibody used in the present invention may be either a polyclonal antibody or a monoclonal antibody as long as the antibody can bind to a nonspecific-reaction causative substance in a fecal sample so as to inhibit nonspecific reaction. The anti-IgA antibody can be prepared in accordance with a common procedure. In addition to the whole molecule of the antibodies, functional fragments of the antibody having an antigen-antibody reaction activity are also regarded as antibodies in the present invention. Examples of the functional fragments of an antibody include F(ab')₂, Fab', etc. These functional fragments can be manufactured by treating the antibody with a proteolytic enzyme (e.g., pepsin or papain). The antibody can be modified before use, and those acquired by binding a polymer compound to the antibody or derivatized by chemical modification can be utilized.

The anti-IgA antibody of the present invention may be present in any state capable of inhibiting nonspecific reaction in a sample and can be added to and used in an immunoreaction system. For example, the antibody may be contained in a sample diluent or, when using immunochromatography, the antibody may be contained in a sample pad, a third pad, a conjugate pad, and an insoluble membrane containing a detection antibody.

When contained in the insoluble membrane, the anti-IgA antibody is desirably located upstream of the part where the detection antibody exists (detecting portion). Among these, the antibody is particularly desirably contained in a sample diluent, a sample pad, or a third pad. When a sample pad and an insoluble membrane are made up of the same pad such that different parts on the same pad are allowed to assume roles of a sample supply portion and a detecting portion, the anti-IgA antibody is desirably present in the sample supply portion.

The anti-IgA antibody may be contained in each device in any amount capable of inhibiting nonspecific reaction and, for example, the content per device may be 10 to 10000 µg/test, preferably 10 to 1000 µg/test, more preferably 25 to 500 µg/test, further preferably 40 to 200 µg/test, and furthermore preferably 40 to 100 µg/test.

To inhibit nonspecific reaction caused in a fecal sample in a more complete manner, in addition to the anti-IgA antibody of the present invention, a combination with another inhibitor of nonspecific reaction is also available within a scope not interfering with the action of the anti-IgA antibody against nonspecific reaction. Examples of such a substance include a so-called commercially available "blocking agent" and antibodies other than anti-IgA antibodies, for example, an anti-IgG antibody, an anti-IgM antibody, an anti-IgD antibody, and an anti-IgE antibody. The blocking agent may appropriately be selected as an agent not affecting a reaction system out of NEO PROTEIN SAVER (Toyobo Enzymes), Immunoblock™ (DS Pharma Biomedical), Applie Block (Seikagaku Biobusiness), SEA BLOCK™/EIA/WB (East Coast Biologics), Blocking One (Nacalai Tesque), bovine serum albumin (hereinafter referred to as "BSA"), Blocking Peptide Fragment (Toyobo Enzymes), Starting Block™ (PBS) Blocking Buffer (Thermo Fisher Scientific), Smart Block™ (CANDOR Bioscience), HeteroBlock (OMEGA Biologicals), etc. Among these, anti-IgM antibody is particularly desirable as the antibody.

Because of discovery of the existence of nonspecific reaction that cannot be inhibited by an anti-IgA antibody alone and an anti-IgM antibody alone and that can be inhibited only when both of the antibodies are allowed to be present, the present inventors have succeeded in widely inhibiting causative substances for the first time by combining both of the antibodies. Examples of the anti-IgM antibody commercially available as a reagent mainly composed thereof include HBR (heterophilic blocking reagent, Scantibodies) (Clinical Chemistry 45:7, 942-956, 1999).

The content of the anti-IgM antibody per device is preferably 5 to 500 µg/test, more preferably 10 to 250 µg/test, further preferably 20 to 100 µg/test, and 40 to 100 µg/test.

### (Sample)

In the present invention, feces are used as a sample. Feces may directly be used as a sample or may appropriately be diluted with a diluent and used as a sample. Feces appropriately diluted and filtered may also be used as a sample.

### (Analyte)

An analyte of the present invention may be any analyte contained in feces as a sample and detectable by using an antigen-antibody reaction, and examples thereof include viruses, parasites, and proteins.

For example, infectious gastroenteritis is mainly caused by viruses and parasites, and examples of viruses as an analyte include norovirus, adenovirus, rotavirus, sapovirus, enterovirus, etc. and examples of parasites as an analyte include Cryptosporidium, amebic dysentery, Giardia, etc.

Additionally, examples of viruses as an analyte include an influenza virus, and examples of proteins as an analyte include human hemoglobin, a hepatitis B virus antibody, a hepatitis C virus antibody, a human immunodeficiency virus antibody, etc.

### (Immunological Detection Method)

An immunological detection method provided by the present invention may be any detection method using an immunoreaction, and examples thereof include a latex turbidimetric immunoassay method (hereinafter referred to as an LTIA method) that is a particle agglutination immunoassay method, an ELISA method that is a typical labeled immunoassay method, a detection method using immunochromatography, etc., and among these, a detection method using immunochromatography is particularly desirable.

### (LTIA Method)

The LTIA method is a method typically using two or more anti-analyte antibodies such that at least one is immobilized on a latex so as to detect an analyte through formation of an immune complex of an analyte in a feces sample and the latex-immobilized antibody and agglutination.

Reagents of the LTIA method used in a clinical examination are generally provided in the form of a first reagent solution and a second reagent solution, and are sequentially mixed with a sample when used.

The anti-IgA antibody of the present invention may be contained in either one or both of the first reagent solution and the second reagent solution and is desirably contained in at least the first reagent solution. When the anti-IgM antibody is included in the present invention, the anti-IgM antibody may be contained in either one or both of the first reagent solution and the second reagent solution and is desirably contained in at least the first reagent solution.

Latex particles used in the LTIA method can appropriately be selected in terms of material, particle diameter, and surface charge amount so as to acquire desired performance such as sensitivity improvement. The latex particles may be any particles suitable for supporting the anti-analyte antibodies. For example, the particles may be made of polystyrene, a styrene-styrene sulfonic acid (salt) copolymer, a styrene-methacrylic acid copolymer, an acrylonitrile-butadiene-styrene copolymer, a vinyl chloride-acrylate ester copolymer, or a vinyl acetate-acrylate ester copolymer. Although the shape of the latex particles is not particularly limited, the average particle diameter thereof is preferably sufficiently large so that agglutinations resulting from an agglutination reaction between the antibody on the surface of the latex particles and the analyte are detectable with the naked eye or optically detectable. A preferable average particle diameter is 0.02 to 1.6 µm, particularly preferably 0.03 to 0.5 µm. Particles made of materials such as metal colloid, gelatin, liposome, microcapsules, silica, alumina, carbon black, a metal compound, metal, ceramics, or a magnetic material are usable instead of the latex particles.

### (ELISA method)

The ELISA method is a method typically using two anti-analyte antibodies such that (a) a sample is added to a solid phase (e.g., a plate) on which a first anti-analyte antibody is immobilized, so as to form a complex between an object (analyte) in the fecal sample and the immobilized antibody and that (b) a sandwich with the complex is formed by a second anti-analyte antibody labeled with a labeling substance and is then detected.

The anti-IgA antibody of the present invention may be contained in a diluent for diluting a sample, may previously be added to the solid phase of (a) and present in a liquid form, may be added and then dried to be present in a dried state until being used, or may be contained in a solution containing the antibody of (b), as long as the anti-IgA antibody is present when the immunoreaction is performed on the solid phase, and among these cases, the anti-IgA antibody is desirably contained in a diluent for diluting a sample.

More specifically, the solid phase (such as a plate) having the first anti-analyte antibody immobilized thereon captures an analyte in a fecal sample to form an analyte-antibody complex. The second anti-analyte antibody labeled with a labeling substance reacts with the analyte-antibody complex to form a sandwich, so that the analyte in the sample can be measured by measuring the amount of the labeling substance with a method appropriate for the labeling substance. For specific methods for constituting measurement reagents (kit) such as a method of immobilizing the first anti-detection-target antibody onto the solid phase and a method of labeling the second anti-analyte antibody with the labeling substance, methods well known to those skilled in the art can be used without limitation in addition to the methods described in this description. Although this configuration can be formed as a homogeneous measurement system, it is preferable to form a heterogeneous measurement system.

### (Detection Method Using Immunochromatography)

In the detection method using immunochromatography, the following test strip is typically used.

The immunochromatographic test strip of the present invention is a test strip for developing a fecal sample in an insoluble membrane so as to detect a complex between an analyte in the sample and a conjugate, and the test strip has the conjugate in which an antibody or antigen immunologically reactive with the analyte is immobilized on a label, and has the following constituents (1) and (2):
(1) a sample pad having a sample supply portion and an anti-IgA antibody retained in an elutable manner; and
(2) an insoluble membrane having a detecting portion in which an antibody or antigen immunologically reactive with the analyte is immobilized for detecting the complex between the analyte in the sample and the conjugate.

The conjugate has an antibody or antigen immunologically reactive with the analyte immobilized on a label and, with regard to the presence form (mode of existence) of the conjugate, the conjugate may be present in a state of being contained in the conjugate pad (type A), may be present as a conjugate portion in a portion of the sample pad (type B), or may be present as an individual conjugate reagent to be mixed with a specimen separately from the test strip (type C).

The test strip having the conjugate in the presence form of the type A will hereinafter be described.

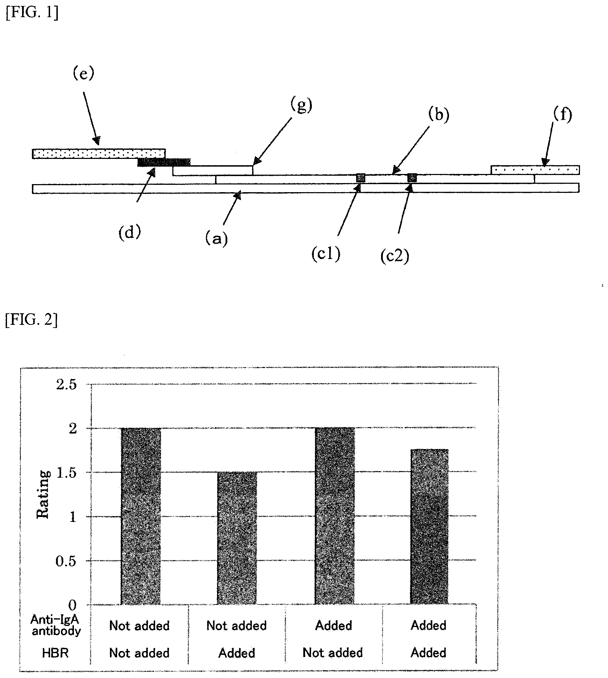

A sample pad, a conjugate pad, a third pad, and an insoluble membrane are arranged in this order from upstream to downstream in the flow direction of the sample and are arranged such that upper and lower layers at least partially overlap with each other. A test strip of such an arrangement example is shown in Fig. 1.

When a sample containing an analyte is supplied to the sample pad of such a test strip, the analyte flows through the sample pad to the conjugate pad on the downstream side. In the conjugate pad, the analyte and the conjugate come into contact with each other and pass through the pad while forming a complex (aggregate). Subsequently, the complex passes through the porous third pad disposed in contact with the lower surface of the conjugate pad and is developed into the insoluble membrane.

Since the insoluble membrane has an antibody or antigen immunologically reactive with the analyte immobilized on a portion thereof, the complex is bound and immobilized onto this membrane due to an immunoreaction. The immobilized complex is detected by a means detecting an absorbance or a reflected light derived from the conjugate.

The test strip having the conjugate in the presence form of the type B will then be described.

A difference from the type A test strip is that the sample pad and the conjugate pad are integrated, i.e., that a sample supply portion and a conjugate portion are formed in portions of the sample pad.

The sample supply portion is a part to which the sample containing analyte is supplied, while the conjugate portion is a part containing the conjugate, and the sample supply portion is located upstream of the conjugate portion.

The test strip having the conjugate in the presence form of the type C will then be described.

The difference from the type A test strip is that the conjugate pad is absent and that the conjugate is present as a separate conjugate reagent. For example, a filter chip may be included that has the conjugate incorporated in a filter. By using such a filter chip and allowing a specimen diluent to pass therethrough, the conjugate and the analyte are bound to form the complex (aggregate). This complex can be supplied to the same test strip as the type A except the absence of the conjugate pad, so as to detect the analyte.

### (Sample Pad)

The sample pad used in the present invention has a part (sample supply portion) receiving a sample. Any substances and forms are available as long as those in a state of being molded into a pad shape can absorb a liquid sample and allow passage of the liquid and the analyte. Specific examples of materials suitable for the sample pad include, but not limited to, glass fiber, acrylic fiber, hydrophilic polyethylene material, dry paper, paper pulp, woven fabric, etc. Preferably, a glass fiber pad is used. The sample pad can also have a function of a conjugate pad described later. The sample pad can also contain a commonly used blocking reagent for the purpose of preventing/inhibiting nonspecific reaction (adsorption) in an antibody-immobilized membrane.

### (Third Pad)

The third pad is desirably disposed as needed depending on properties etc. of the sample and may be any pad as long as the pad can allow passage of the complex between the analyte in the sample and the conjugate. Specifically, although examples include glass fiber, acrylic fiber, hydrophilic polyethylene material, dry paper, paper pulp, woven fabric, etc., the pad is particularly desirably a porous member made of polysulfone or cellulose acetate.

The pore diameter of the porous third pad of the present invention is desirably 1 to 100 µm, more desirably 10 to 100 µm, still more desirably 20 to 80 µm, most preferably 25 to 70 µm in average pore diameter. This is because a diameter less than 1 µm causes clogging making a specimen flow itself poor, while a diameter greater than 100 µm appears to make it unable to capture the nonspecific reaction substance.

### (Insoluble Membrane)

The insoluble membrane used in the present invention has at least one detecting portion on which an antibody or antigen immunologically reactive with the analyte is immobilized. The antibody or antigen immunologically reactive with the analyte can be immobilized onto an insoluble membrane carrier by a conventionally known method. In the case of a lateral flow immunochromatography reagent, after a solution containing a predetermined concentration of the antibody or the antigen is prepared, the solution is applied in a line shape to the insoluble membrane carrier by using an apparatus etc. having a mechanism capable of moving a nozzle in a horizontal direction while discharging the solution at a constant rate therefrom, and is dried for immobilization. The concentration of the antibody or the antigen in the solution is preferably 0.1 to 5 mg/mL, more preferably 0.5 to 2 mg/mL. An amount of the antibody or the antigen immobilized on the insoluble membrane carrier can be optimized by adjusting a discharge rate from the nozzle of the apparatus in the case of the lateral flow type, and is preferably 0.5 to 2 µL/cm.

A measurement method using the lateral flow immunochromatographic reagent is a measurement method in which a sample moves in a direction lateral to the insoluble membrane carrier due to capillarity.

The solution containing the antibody or the antigen at a predetermined concentration can be prepared by adding the antibody or the antigen to a buffer solution. A type of the buffer solution may be a commonly used buffer solution such as a phosphate buffer solution, a Tris buffer solution, and a Good's buffer solution. The buffer solution preferably has pH in a range of 6.0 to 9.5, more preferably 6.5 to 8.5, further preferably 7.0 to 8.0. The buffer solution may further contain salts such as sodium chloride, a stabilizer such as sucrose and a preservative, and an antiseptic such as Proclin (registered trademark). The salts include those contained for adjustment of ionic strength, such as sodium chloride, as well as those added for the purpose of adjustment of pH of the buffer solution, such as sodium hydroxide.

After the antibody or the antigen is immobilized on the insoluble membrane, the insoluble membrane can be coated for blocking with a commonly used blocking agent in a form of solution or vapor, except the part in which the antibody or the antigen is immobilized.

A control capture reagent conventionally used for an immunochromatographic reagent may be immobilized on the insoluble membrane. The control capture reagent is a reagent for ensuring the reliability of assay and captures a control reagent contained in the conjugate pad. For example, if labeled keyhole-limpet haemocyanin (hereinafter referred to as KLH) is contained as a control reagent in the conjugate pad, an anti-KLH antibody etc. correspond to the control capture reagent. A position of immobilization of the control capture reagent can appropriately be selected in accordance with design of an assay system.

A membrane making up the insoluble membrane used in the present invention can be a known membrane conventionally used as an insoluble membrane carrier of an immunochromatographic reagent. For example, the membrane may be made of fibers of polyethylene, polyethylene terephthalate, nylons, glass, polysaccharide such as cellulose and cellulose derivatives, ceramics, etc. Specifically, the membrane may be glass fiber filter paper, cellulose filter paper, etc., commercially available from Sartorius, Millipore, Toyo Roshi, Whatman, etc. In particular, UniSart CN140 from Sartorius is preferable. By selecting a pore diameter and a structure of the insoluble membrane carrier as needed, a rate of flow in the insoluble membrane carrier of the complex between the conjugate and the analyte in the sample can be controlled.

The immunochromatographic test strip is preferably disposed on a solid phase support such as a plastic adhesive sheet. The solid phase support is made of a material not hindering the capillary flow of the sample and the conjugate. The immunochromatographic test strip may be fixed to the solid phase support with an adhesive etc. In this case, an adhesive component etc., are also made of a material not hindering the capillary flow of the sample and the conjugate. The immunochromatographic test strip can be used after installed in or mounted on an appropriate container (housing) with consideration given to the size of the immunochromatographic test strip, the method and the position of addition of the sample, the position of formation of the detecting portion of the insoluble membrane carrier, the signal detection method, etc., and such an installed/mounted state is referred to as a "device".

### (Antibody or Antigen immunologically Reactive with Analyte)

The antibody or antigen immunologically reactive with an analyte used in the present invention is an antibody or antigen capable of binding to the analyte and is preferably an antibody when the analyte is a virus or an antigen, or is an antigen when the analyte is an antibody. The antibodies or antigens immunologically reactive with the analyte are immobilized on the label described later and the detecting portion. Although the antibodies or the antigens immobilized on the label and the detecting portion may be the same, the antibodies or the antigens for the label and for the detecting portion are preferably different.

### (Label)

For the label used in the present invention, a known label conventionally used for an immunochromatographic test strip can be used. For example, the label is preferably colloidal metal particles such as gold colloid particles and platinum colloid particles, colored latex particles, magnetic particles, fluorescent particles, etc. and is particularly preferably gold colloid particles and colored latex particles.

### (Conjugate)

The conjugate used in the present invention is the label as described above with the antibody or antigen immunologically reactive with the analyte immobilized thereon. When norovirus is detected as the analyte, preferably, an anti-norovirus monoclonal antibody is immobilized on gold colloid particles.

Examples of a method of immobilizing an antibody or antigen immunologically reactive with an analyte onto a label include physical adsorption, chemical bonding, etc., and immobilization is typically achieved by physical adsorption.

### (Others)

The immunochromatographic test strip of the present invention may further include other reagents and constituents depending on the measurement condition and the sample.

Other reagents include a blocking agent preventing nonspecific reaction, for example.

Other constituents include an absorption pad absorbing the sample having moved/passed through the insoluble membrane to control the development of the sample, for example.

The immunochromatographic test strip of the present invention can be produced in accordance with the methods described in Examples with modifications/alterations made as needed.

### (Kit)

A detection kit using immunochromatography of the present invention may be any kit containing the immunochromatographic test strip described above. The detection kit may also include other reagents necessary for detection, a diluent for a sample, a test tube, a cotton swab for fecal sampling, an instruction manual, a housing for installing the test strip, etc.

### (Others)

In this description, the terms upstream or downstream is used to mean the upstream side or the downstream side in the flow direction of the sample. Therefore, when the test strip of the present invention has a sample pad, a conjugate pad, a third pad, and an insoluble membrane laminated from the top in a partially overlapping manner, the sample pad is on the most upstream side and the insoluble membrane is on the most downstream side. An end pad may be laminated on the upper side overlapping with a downstream end portion of the insoluble membrane and, in this case, the end pad is on the most downstream side.

### EXAMPLES

### [Example 1] Screening of Inhibitor of Nonspecific Reaction

A nonspecific reaction-inhibition effect of an anti-IgA antibody was examined together with blocking agents and surfactants generally known as inhibitors of nonspecific reaction.

### 1. Production of immunochromatographic test strips

### 1) Preparation of gold colloid-labeled anti-norovirus monoclonal antibody (anti-norovirus antibody conjugate)

### (i) Preparation of gold colloid solution

To 5,000 mL of purified water heated to 80 °C, 10 mL each of a 5.0% triammonium citrate aqueous solution and a 5.0% tetrachloroauric (III) acid aqueous solution was added and reacted for 10 minutes with stirring. Subsequently, the reaction solution was heated again and, after 35 minutes, cooled in ice water to produce a gold colloid solution having an average particle diameter of 50 nm. The colloidal gold solution was adjusted with RO water to a gold colloid solution (pH 8.5) having an average particle diameter of 50 nm with the absorbance at the local maximum absorption wavelength of 1.0 OD/mL.

### (ii) Preparation of anti-norovirus antibody conjugate

To 20 mL of the gold colloid solution (pH 8.5) acquired in (i) described above, 1.0 mL of a 2 mmol/L boric acid solution (pH 8.5) containing a 50 µg/mL anti-norovirus antibody (Cosmo Bio) was added and stirred for 10 minutes. Subsequently, 1.5 mL of 10% BSA was added, and further stirred for 5 minutes to prepare an antibody-sensitized gold colloid solution. Centrifugation of the antibody-sensitized gold colloid solution was performed at 10 °C for 45 minutes at 10,000 rpm and followed by removal of the supernatant, and the antibody-sensitized gold colloid precipitated by the centrifugation was diluted with a conjugate diluent (conjugate dilution buffer, SCRIPPS) and collected. The collected antibody-sensitized gold colloid was diluted with a conjugate diluent containing 0.5% casein to the absorbance at the local maximum absorption wavelength of 1.5 O.D./mL to prepare a conjugate pad application solution (conjugate pad application solution 1).

Instead of 10% BSA, another blocking agent BPF (Blocking Peptide Fragment) (BPF-301 manufactured by Toyobo Enzymes) or NPS (Neo Protein Saver) (NPS-301 manufactured by Toyobo Enzymes) was added at the same concentration to produce an antibody-sensitized gold colloid solution in the same way (conjugate pad application solutions 2, 3).

Moreover, surfactants Epan U108 (manufactured by DKS), Epan 485 (manufactured by DKS), Pluronic F68 (manufactured by ADEKA) were added to the conjugate pad application solution 1 at 1%, 0.1%, 0.05%, respectively, to prepare solutions (conjugate pad application solutions 4, 5, 6).

### (iii) Preparation of gold colloid-labeled KLH (KLH conjugate) for control line

To 20 mL of the 1.0 OD/mL gold colloid solution above (pH 6.1), 1 mL of KLH (manufactured by Sigma) dissolved at 620 µg/mL with a 2 mmol/L phosphate buffer solution (pH 6.1) was added and stirred at room temperature for 10 minutes. To the mixed solution of the gold colloid and KLH, 1 mL of a 10% BSA aqueous solution was added and stirred at room temperature for 5 minutes.

Subsequently, the mixture was centrifuged at 10 °C for 45 minutes at 11900×g. After removing the supernatant, 1 mL of the conjugate diluent above was added to the acquired sediment to suspend the conjugate so as to acquire a KLH conjugate.

### 2) Preparation of conjugate pad

The anti-norovirus antibody conjugate pad application solutions prepared in 1) described above and the 3 OD/mL KLH conjugate were each mixed with a 1.33% casein, 4% sucrose solution (pH 7.5) to prepare conjugate solutions, and glass fiber pads (Millipore) having a constant volume were impregnated with 1.13 parts by volume of the solutions in terms of the pad volume. The pads were dried by heating in a dry oven at 70 °C for 45 minutes to acquire conjugate pads.

### 3) Preparation of anti-norovirus monoclonal antibody-immobilized membrane (insoluble membrane)

An anti-norovirus antibody (Cosmo Bio) different in epitope from the labeled antibody was prepared at 0.75 mg/mL with a 10 mmol/L phosphate buffer (pH 7.2) containing 2.5% sucrose to acquire a test line application solution. An anti-KLH antibody (derived from rabbit) was prepared in the same way at 0.75 mg/mL with the 10 mmol/L phosphate buffer (pH 7.2) containing 2.5% sucrose to acquire a control line application solution.

The test line application solution and the control line application solution were applied to a nitrocellulose membrane (manufactured by Sartorius) in the shape of 1-mm-wide lines with an interval in between. The application was performed by using an immunochromatographic dispenser "XYZ3050" (manufactured by BIO DOT) with the application amount set to 1.0 µL/cm. The membrane was dried by heating in a dry oven at 70 °C for 45 minutes to prepare an antibody-solid-phased membrane.

### 4) Preparation of sample pad

Twenty (20) mmol/L tris buffer (pH 7.2) containing 0.5% sucrose and 250 mmol/L NaCl was prepared as a sample pad application solution (sample pad application solution 1).

An anti-IgA antibody (Cosmo Bio) was added at 1.5 mg/mL (90.7 mg/test) to the sample pad application solution 1 to prepare a solution (sample pad application solution 2).

To a glass fiber pad (manufactured by Lydall) having a constant volume, the sample pad application solution 1 or the sample pad application solution 2 was applied at 2.58 parts by volume in terms of the pad and was dried by heating in a dry oven at 70 °C for 45 minutes to acquire each sample pad.

### 5) Assembly of immunochromatographic test strip

Fig. 1 shows a schematic configuration diagram of the immunochromatographic test strip of the present invention.

An insoluble membrane (b) was affixed to a plastic adhesive sheet (a), and a third pad (manufactured by Pall) (g), a conjugate pad (d), and a sample pad (e) were then sequentially disposed and mounted along with an absorption pad (manufactured by Whatman) (f) disposed and mounted on the end on the opposite side. The conjugate pad was impregnated with a conjugate having gold colloid sensitized with an anti-norovirus antibody, and the insoluble membrane had an anti-norovirus antibody (c1) and a control reagent (c2) immobilized on lines perpendicular to the flow direction such that a test line was on the upstream side in the flow direction. The pads were each laminated and arranged with a portion thereof brought into contact with upper and lower pads. The line comprising the anti-norovirus antibody (c1) is referred to as a test line, and the line comprising the control reagent (c2) is referred to as a control line. For the third pad, a porous membrane made of polysulphone and having an average pore size of 20 to 50 µm was used. A structure acquired by overlapping the constituent elements in this way was cut by a constant width to produce immunochromatographic test strips. The test strips can each be installed and mounted in a dedicated plastic housing to achieve a form of an immunochromatographic test device (not shown).

### 2. Test procedure

Feces of nine normal individuals were used as specimens (specimens are all norovirus-free, i.e. negative, specimens). Each of the specimens was suspended to 0.1 g/mL in a 20 mmol/L phosphate buffer solution (pH 7.6) containing a surfactant, thoroughly stirred by vortexing, and then centrifuged at 3000 rpm for 10 minutes to acquire a supernatant, which was used as a sample.

To the various test strips produced in 1. described above, 120 µL of the sample was added dropwise and, the presence or absence of false positive was visually observed after 15 minutes.

### 3. Test results

The observation results are shown in Table 1. A circle indicates the case that the nonspecific reaction-inhibition effect was provided, and a cross indicates the case that no effect was provided. When a commercially available blocking agent or surfactant was added, the nonspecific reaction-inhibition effect was not observed in each case and a false positive was observed. On the other hand, when the anti-IgA antibody was added, nonspecific reaction was inhibited.

**[Table 1]**

| | Conjugate pad application solution No. | Sample pad application solution No. | Substance added as candidate for inhibitor of nonspecific reaction | Nonspecific reaction-inhibition effect |
|---|---|---|---|---|
| Test strip 1 | 1 | 1 | BSA | × |
| Test strip 2 | 2 | 1 | BPF | × |
| Test strip 3 | 3 | 1 | NPS | × |
| Test strip 4 | 4 | 1 | BSA, EpanU108 | × |
| Test strip 5 | 5 | 1 | BSA, Epan 485 | × |
| Test strip 6 | 6 | 1 | BSA, Pluronic F68 | × |
| Test strip 7 | 1 | 2 | BSA, anti-IgA antibody | ○ |

### [Example 2] Confirmation Test for Nonspecific Reaction-Inhibition Effect of Anti-IgA Antibody

### 1. Production of immunochromatographic test strips

Test strips were produced in the same way as Example 1 by using the following (1) to (4) as the sample pad application solutions and the conjugate pad application solution 1 of Example 1 as the conjugate pad application solution. It is noted that HBR is an inhibitor of nonspecific reaction having an anti-IgM antibody as an active ingredient (Clinical Chemistry 45:7, 942-956, 1999):
(1) the sample pad application solution 1 of Example 1;
(2) the sample pad application solution 1 of Example 1 to which 0.75 mg/mL (45.4 µg/test) of HBR (manufactured by Scantibody) was added;
(3) the sample pad application solution 1 of Example 1 to which 1.5 mg/mL (90.7 µg/test) of an anti-IgA antibody (derived from goat) was added; and
(4) the sample pad application solution 1 of Example 1 to which 1.5 mg/mL (90.7 µg/test) of an anti-IgA antibody (derived from goat) and 0.75 mg/mL (45.4 µg/test) of HBR were added.

### 2. Test Procedure

Feces of 42 normal individuals were used as specimens (specimens are all norovirus-free, i.e. negative, specimens). Each of the specimens was suspended at 0.1 g/mL in a 20 mmol/L phosphate buffer solution (pH 7.6) containing a surfactant, thoroughly stirred by vortexing, and then centrifuged at 3000 rpm for 10 minutes to acquire a supernatant, which was used as a sample.

To the test strips produced in 1. described above, 120 µL of the sample was added dropwise and, the presence or absence of false positive was visually observed after 10, 15, and 30 minutes. From the visual observation results, the coloring intensity of the test line was quantified by using a color sample called "color chart". The color intensity on the color chart was quantified as values from 0 to 4 in increments of 0.25. From the evaluation result of the coloring intensity, the strength of nonspecific reaction was evaluated.

Evaluation criteria are as follows:
++: strong nonspecific reaction exists (1.0 or more);
+: weak nonspecific reaction exists (0.25 or more and less than 1.0); and
-: no nonspecific reaction exists (0).

### [Table 2]

**Table 2-1**

| | specimen number | 1 | 2 | 3 | 4 | 5 | 6 | 7 |
|---|---|---|---|---|---|---|---|---|
| (1) Anti -immunoglobulin antibody not added | 10min | - | - | + | + | - | + | ++ |
| | 15min | - | + | ++ | ++ | - | + | ++ |
| | 30min | + | + | ++ | ++ | + | ++ | ++ |
| (2) Only HBR (anti-IgM antibody) added | 10min | - | - | - | - | - | - | - |
| | 15min | - | + | - | - | - | - | - |
| | 30min | + | + | + | - | - | - | - |
| (3) Only anti-IgA antibody added | 10min | - | - | - | - | - | + | ++ |
| | 15min | - | - | - | + | - | + | ++ |
| | 30min | - | - | - | + | + | + | ++ |
| (4) Both HBR and anti-IgA antibody added | 10min | - | - | - | - | - | - | - |
| | 15min | - | - | - | - | - | - | - |
| | 30min | - | - | - | - | - | - | - |

**Table 2-2**

| | specimen number | 8 | 9 | 10 | 11 | 12 | 13 | 14 | 15 | 16 | 17 |
|---|---|---|---|---|---|---|---|---|---|---|---|
| (1) Anti -immunoglobulin antibody not added | 10min | - | - | - | - | ++ | + | - | - | - | - |
| | 15min | - | - | - | + | ++ | ++ | - | + | - | + |
| | 30min | + | + | + | + | ++ | ++ | + | ++ | + | + |
| (2) Only HBR (anti-IgM antibody) added | 10min | - | - | - | - | - | - | - | - | - | - |
| | 15min, | - | - | - | - | - | - | - | - | - | - |
| | 30min | - | - | - | - | - | - | - | - | + | + |
| (3) Only anti-IgA antibody added | 10min | - | - | - | - | - | - | - | - | - | - |
| | 15min | - | - | - | - | - | - | - | - | - | + |
| | 30min | - | - | - | - | - | - | - | - | + | + |
| (4) Both HBR and anti-IgA antibody added | 10min | - | - | - | - | - | - | - | - | - | - |
| | 15min | - | - | - | - | - | - | - | - | - | - |
| | 30min | - | - | - | - | - | - | - | - | - | - |

### 3. Test results

### (1) Nonspecific reaction-inhibition effect

Among the 42 specimens, even when the anti-IgA antibody was not added, nonspecific reaction was not observed in some specimens (i.e., negative specimens) (the results are not shown in the table). The remaining 17 specimens other than these negative specimens were specimens (false positive specimens) in which nonspecific reaction occurs when the anti-IgA antibody was not added. For these false positive specimens (specimen Nos. 1 to 17), the nonspecific reaction-inhibition effect was examined when only HBR (main component: anti-IgM antibody) was added, when only the anti-IgA antibody was added, and when both HBR and the anti-IgA antibody were added, and the results are shown in Table 2.

For specimen Nos. 1 to 3, it can be seen that the addition of the anti-IgA antibody was effective, while the addition of HBR did not inhibit nonspecific reaction.

For specimen Nos. 4 to 7, it can be seen that the addition of HBR has the nonspecific reaction-inhibition effect, while the addition of the anti-IgA antibody did not inhibit nonspecific reaction.

For specimen Nos. 8 to 15, it can be seen that nonspecific reaction was inhibited when either HBR or the anti-IgA antibody was added.

For specimen Nos. 16 and 17, it can be seen that no effect was observed when only HBR or only the anti-IgA antibody was added and that nonspecific reaction was inhibited only when both were added.

From the above, as was the case with commercially available HBR, the anti-IgA antibody alone was effective for about 70% of the false positive specimens, and the combination of both was able to inhibit nonspecific reaction in almost all the false positive specimens.

### [Example 3] Confirmation Test for Influence on Virus Detection Sensitivity

The virus detection sensitivity according to addition of the anti-IgA antibody was examined. The case of adding the commercially available nonspecific reaction inhibitor HBR was also examined as a reference example.

### 1. Production of immunochromatographic test strip

The production was the same as Example 2.

### 2. Test procedure

A norovirus positive control was diluted to an arbitrary concentration with saline. A sample solution was prepared by adding 10 µL of the diluted Norovirus positive control to 1,000 µL of a specimen diluent.

To the test strips produced in 1. described above, 120 µL of the sample solution was added dropwise and, the line intensity in the test line after 15 minutes was quantified and evaluated by using the color chart.

### 3. Test results

The results are shown in Fig. 2. A reduction in sensitivity to the norovirus-positive control was not observed in those to which the anti-IgA antibody was added. On the other hand, a reduction in detection sensitivity was observed in the reference example to which HBR was added.

From the above, it was found that the anti-IgA antibody has extremely high practicality as an inhibitor of nonspecific reaction.

### INDUSTRIAL APPLICABILITY

By performing an immunoreaction in the presence of an anti-IgA antibody according to the present invention, nonspecific reaction caused by a fecal sample can be inhibited so as to accurately detect an analyte in a sample. Particularly, by using immunochromatography as the immunoreaction, the analyte in the fecal sample can easily and accurately be detected.

Additionally, combination with an anti-IgM antibody can inhibit nonspecific reaction with a very high likelihood without causing a reduction in detection sensitivity, so that a very useful immunological detection method can be provided.

### REFERENCE SIGNS LIST

(a) Plastic adhesive sheet
(b) Insoluble membrane
(c1) Anti-norovirus antibody
(c2) Control reagent
(d) Conjugate pad
(e) Sample pad
(f) Absorption pad
(g) Third pad

## Claims

1. A method of immunologically detecting an analyte in a fecal sample, wherein an immunoreaction is performed in the presence of an anti-IgA antibody.

2. The method according to claim 1, wherein the immunoreaction is performed in the presence of the anti-IgA antibody and an anti-IgM antibody.

3. The method according to claim 1, wherein the method is a detection method using immunochromatography developing the fecal sample in an insoluble membrane so as to detect a complex between the analyte in the sample and a conjugate, and wherein the immunological detection method includes the steps of:
(a) bringing the sample into contact in the presence of the anti-IgA antibody with the conjugate in which an antibody or antigen immunologically reactive with the analyte is immobilized on a label, so as to form the complex between the analyte in the sample and the conjugate; and
(b) developing the complex in the insoluble membrane so as to detect the complex in a detecting portion in which an antibody or antigen immunologically reactive with the analyte is immobilized.

4. The method according to claim 3, wherein the step of (a) above is a step of bringing the sample into contact in the presence of both the anti-IgA antibody and an anti-IgM antibody with the conjugate in which an antibody or antigen immunologically reactive with the analyte is immobilized on a label, so as to form the complex between the analyte in the sample and the conjugate.

5. A method of inhibiting nonspecific reaction in a method of immunologically detecting an analyte in a fecal sample, wherein an immunoreaction is performed in the presence of an anti-IgA antibody.

6. The method according to claim 5, wherein the immunoreaction is performed in the presence of the anti-IgA antibody and an anti-IgM antibody.

7. The method according to claim 5, wherein the method is a method of inhibiting nonspecific reaction in detection using immunochromatography developing a fecal sample in an insoluble membrane so as to detect a complex between the analyte in the sample and a conjugate, and wherein the immunological detection method includes the steps of:
(a) bringing the sample into contact in the presence of the anti-IgA antibody with the conjugate in which an antibody or antigen immunologically reactive with the analyte is immobilized on a label, so as to form the complex between the analyte in the sample and the conjugate; and
(b) developing the complex in the insoluble membrane so as to detect the complex in a detecting portion in which an antibody or antigen immunologically reactive with the analyte is immobilized.

8. The method according to claim 7, wherein the step of (a) above is a step of bringing the sample into contact in the presence of both the anti-IgA antibody and an anti-IgM antibody with the conjugate in which an antibody or antigen immunologically reactive with the analyte is immobilized on a label, so as to form the complex between the analyte in the sample and the conjugate.

9. An immunochromatographic test strip for developing a fecal sample in an insoluble membrane and detecting a complex between an analyte in the sample and a conjugate, the test strip having the conjugate in which an antibody or antigen immunologically reactive with the analyte is immobilized on a label, the test strip comprising:
(1) a sample pad having a sample supply portion and an anti-IgA antibody retained in an elutable manner; and
(2) an insoluble membrane having a detecting portion on which an antibody or antigen immunologically reactive with the analyte is immobilized for detecting the complex between the analyte in the sample and the conjugate.

10. The test strip according to claim 9, comprising a conjugate pad having the conjugate retained in an elutable manner.

11. The test strip according to claim 9 or 10, wherein the sample pad further has an anti-IgM antibody retained in an elutable manner.

12. An inhibitor for a fecal sample of nonspecific reaction in an immunoreaction comprising an anti-IgA antibody as an active ingredient.

13. The inhibitor of nonspecific reaction according to claim 12, further comprising an anti-IgM antibody.
